# EUROPEAN PATENT APPLICATION

(11) **EP 3 281 695 A1**
(43) Date of publication of application: **14.02.2018**
(21) Application number: 16183713.3
(22) Date of filing: 11.08.2016
(51) Int. Cl.: B01J 20/02, B01J 20/22, B01J 20/28, B01J 20/282, B01J 20/286, B01J 20/289, B01J 20/30, B01J 20/32, B01J 20/20

(54) **FILTRATION DEVICE**

(71) Applicant: Freie Universität Berlin, 14195 Berlin (DE); Largentec Gmbh, 14129 Berlin (DE)
(72) Inventor: LANDAU, Uwe, 14169 Berlin (DE); GROHMANN, Elisabeth, 14052 Berlin (DE); HAAG, Rainer, 12209 Berlin (DE); QI, Zhenhui, 14109 Berlin (DE)
(74) Representative: Remus, Alvaro Johannes

(57) **Abstract**

The invention relates to a filtration device 1 comprising at least one active layer 2 and at least one antimicrobial composite 3. The invention further concerns a method for producing a filtration device and filter systems comprising at least two substrates or components. The filtration device 1 may comprise two substrates, substrate A being provided with a chemically functionalized graphene or graphene oxide layer (active layer 2) and substrate B being coated with an antimicrobial composite 3 comprising silver and ruthenium ("AGXX"). The active layer 2 comprises graphene or graphene oxide functionalized by ionic groups so that it can attract microorganisms 4 and endotoxins (Step I), which are then captured and bound by the active layer 2 (Step II). The antimicrobial composite 3 produces and releases reactive oxygen species ("ROS") which kill microorganisms present in the surroundings (Step III). As the cell structure of the microorganisms is destroyed during the killing process, the cell debris is at least in part removed from the active layer 2 (Step IV).

## Description

The invention relates to a filtration device comprising at least one active layer and at least one antimicrobial composite. The invention further concerns a method for producing a filtration device and filter systems comprising at least two substrates or components.

### Background of the invention

Outbreaks of infectious diseases like SARS (2003, 774 deaths), Swine flu (2009, 14,286 deaths) and Ebola (2014, 10,194 deaths) are unavoidable natural events which go hand in hand with indescribable personal tragedy and significant economic losses. Globalization further accelerates the world-wide spreading of lethal diseases and challenges the healthcare system to provide protection and credibility to its customers. The major tool of the healthcare system, i.e., treatment of diseases with drugs, is becoming less efficient and economically feasible as the long-term treatment of patients results in an ever increasing number of drug-resistant germs, i.e., bacteria or viruses. Only in Germany, each year up to 15,000 people die by these man-made "hospital germs". A recent European survey which monitored more than 1,000 hospitals in 30 European countries, further revealed that on any given day, about 80,000 patients in European hospitals have at least one healthcare-associated infection.

Therefore, it is not surprising that many authorities like World Health Organization (WHO) and European Disease Prevention and Control (ECDC) have made strong recommendations for developing and implementing novel infection control strategies and technologies. Pathogens, like bacteria and viruses, are either transported through the air, water or by contact with body fluids, whereby previous experience has shown that medical surgical barrier materials, i.e., surgical masks or "non-wovens" (gloves, gowns and drapes), could provide a suitable platform to stop their spreading if equipped with the ability to capture and kill pathogens.

### Prior art

DE 44 16 501 A1 discloses a surface filter with gauze filter insert for filtering small solid particles from drinking water, wherein the entire surface of the gauze filter insert is provided with a firmly adhering, poorly water-soluble coating having a germicidal effect. This germicidal coating of the filter inhibits or prevents carrier-fixed growth of bacteria on the surface. In particular, the entire surface of the gauze filter forming materials, i.e. the warp and weft threads of a sieve fabric or the fibers of a nonwoven fabric sieve, are coated with the anti-germ coating. The coating may be made of a heavy metal, preferably silver.

US 8 925 736 B2 discloses a filter that is modified by a polymer-carbon based nanomaterial nanocomposite intended to significantly enhance the performance of filtration, separation, and remediation of a broad variety of chemicals, heavy metal ions, organic matters, and living organisms. Polymeric materials are combined with graphene and graphene oxide chemically modified with a chelating agent such as EDTA. The nanocomposite is homogenously deposited on the surface of a membrane.

US 5 762 797 A discloses an antimicrobial filter cartridge for a water filtration system. The filter cartridge has a perforated core member wrapped with a microporous membrane, which is overwrapped with a spiral wrapping of an antimicrobial agent impregnated yarn. Fluid passing through the cartridge housing is filtered by the filter cartridge to remove microorganisms from the water and prevent the growth of microorganisms on the filter media. The filter cartridge includes an inner tubular-shaped perforated core of a metal, plastic or ceramic material, or formed from activated carbon. The core is covered with a microporous membrane that is tightly wrapped around the core. As antimicrobial agent 2,4,4'-trichloro-2'-hydroxy diphenol ether or 5-chloro-2-phenol (2,4 dichlorophenoxy) is generally used.

US 2013/0330833 A1 discloses a system and device for filtering fluids using graphene oxide, wherein graphene oxide-based filters are used for removal of microorganisms from organic and aqueous liquids, to prevent fuel biodeterioration, and to remove small amounts of water from hydrocarbon fuels. Graphene oxide is functionalized with reactive oxygen functional groups so as to provide a large surface area which can be decorated with antimicrobial agents including nanosilver. The graphene oxide filter media are made of a plurality of graphene oxide particles, a structural core coated with graphene oxide, a non-porous structural membrane coated with graphene oxide, or a filtering membrane coated with graphene oxide.

Surgical masks or "non-wovens" (gloves, gowns and drapes) can be equipped with the ability to capture and kill pathogens, e.g., by the incorporation of noble-metals, i.e., copper and silver, in the form of nanoparticles. However, so far, the high material costs as well as government regulation on the use of nanoparticles have limited a wide-spread application. The current European legislation on biocides limit the application of nanoparticles, in particular the silver nanoparticles, due to their potential danger to human health.

Moreover, noble metal powders, i.e., copper and silver powder, as well as active carbon can be used to provide such surgical barrier materials with antimicrobial properties. Noble metals powders also have the proven ability to have anti-microbial activity, however, they are generally more effective against bacteria but not as effective against viruses. Furthermore, coating materials with noble metal powders are susceptible to the very high and volatile raw material prices of copper and silver. Another common material which is used for filtration devices such as facial masks is active carbon. However, even though it is widely employed because of its low cost and excellent ability to bind hydrophobic chemicals like toxic gases, it is widely ineffective for the binding of pathogens.

In summary, new European standards and regulations force producers of facial masks and non-woven products for medical applications to incorporate anti-microbial properties. Moreover, elimination of infectious microorganisms from water and air as well as water and air-pipes is also a requirement that has to be addressed. Current solutions to comply with the regulations are, however, not cost-effective and their large-scale application is limited by government regulations.

It is therefore the object of the invention to provide a novel filtration device for capturing and killing pathogens from air and water, which is cost-effective, complies with European standards and regulations, and allows large-scale application.

### Summary of the invention

According to the invention the filtration device comprises at least one active layer comprising at least one of graphene and graphene oxide, and at least one antimicrobial composite comprising metallic silver (Ag) and metallic ruthenium (Ru). The filtration device according to the invention can be or at least be part of a particle filter or a fabric (woven) filter for water or air filtration. Moreover, the filtration device according to the invention can be easily incorporated into single-use technology products such as medical non-wovens and surgical masks to efficiently capture and kill pathogens under economically feasible conditions. The filtration device complies with European standards and regulations including EN 13795 and has the required anti-microbial properties. In particular, the filtration device according to the invention is based on 2D carbon sheets comprising graphene and/or graphene oxide and a composite comprising metallic silver and metallic ruthenium. The carbon sheets of the filtration device have an extremely high surface area and can bind to a large variety of germs and pathogens, i.e., influenza virus (H1N1), vesicular stomatitis virus (VSV), respiratory syncytial virus (RSV), herpes simplex virus (HSV) and *Mycobacterium tuberculosis,* with extremely high affinity, similar to the attachment of two sticky stripes of a hook-and-loop fastener. Because of its high binding affinity, germs which have been in contact with the carbon material cannot be released anymore. The anti-microbial effect is provided by the antimicrobial composite, wherein metallic silver (Ag) and metallic ruthenium (Ru) interact such that a microelectric field is generated that results in a redox system causing oxidative death of micro-organisms. That is, the antimicrobial composite leads to fast and efficient killing of microorganisms. Simultaneously, the antimicrobial composite prevents infestation by microorganisms and attachment or permanent deposition of biomolecules such as DNA, RNA, or proteins. A self-cleaning device is thus provided, which very quickly and efficiently and over extended periods of time sterilizes fluids when they come in contact with said device. Moreover, the antimicrobial composite is a very efficient biofilm reductor nearly independent of biofilm forming species. As a result, the filtration device according to the invention allows for effective capturing (by the active layer) and killing (by the antimicrobial composite) of microorganisms.

Besides air transmission water is one of the most dangerous paths for infectious microorganisms. One of the biggest problems for infection control is biofilm formation within pipes for air and water transfer, as microorganisms are steadily released from biofilms. Thus, they are a big source for infection spread. The filtration device according to the invention provides an effective solution by installing it at the pipe outlet, thus microorganisms released from biofilm will be captured and killed before getting into contact with human beings and animals. The filtration device according to the invention may further comprise at least one substrate as a carrier for at least one of the active layer and the antimicrobial composite. For example, the active layer and/or the antimicrobial composite may be disposed on at least a part of the substrate. Alternatively, the antimicrobial composite may be disposed on at least a part of at least one of the substrate and the active layer.

In an advantageous and exemplary embodiment of the invention the substrate is a porous substrate and/or comprises at least one material selected from the group consisting of a fabric, a polymer, a ceramic, and a metal. Preferably, the fabric is a non-woven fabric. The active layer comprising graphene and/or graphene oxide can be easily coated on a fibrous or non-woven matrix, i.e., cotton filters, through simple dip-soaking or spray-coating procedures. For example, cellulose filter paper can be coated with a chemically modified and/or functionalized graphene oxide ("GOX") using diisocyanate (e.g., hexamethyldiisocyanate, isophoronediisocyanate, or methylene diphenyl diisocyanate). To this end, the filter paper is dispersed in a GOX solution, diisocyanate is added, and the mixture is then stirred for several minutes at elevated temperature. Methods for providing a cellulose material with graphene oxide have been published by Kafy et al. (2015) and Sadasivuni et al. (2015). The resulting carbon/fabric hybrids can be rapidly incorporated into current production chains of non-woven products. Additionally, graphene oxide (e.g., "GOX") can be easily cross-linked and become a porous matrix itself without a coating procedure. Microorganisms can be efficiently retained by the porous substrate and then captured and killed by the active layer and the antimicrobial composite, respectively.

In an advantageous embodiment of the invention at least one of the active layer and the antimicrobial composite can be disposed on at least a part of the substrate. That is, the substrate can be coated with the active layer and/or the antimicrobial composite such that microorganisms contacting the device according to the invention can be captured and killed by the coating.

The substrate may be, e.g., at least part of at least one device selected from the group consisting of a membrane, a sieve, a mesh, a mask, a gown, a glove, a drape, a mat, a mat of fibers, and a fleece. Accordingly, the filtration device according to the invention can be, e.g., a filtration membrane, an air or water filter, or a medical non-woven product. In any case, the substrate is designed to filter a fluid such as water or air, wherein the fluid can pass the substrate while particles such as dirt and micro-organisms are held back at its surface. The substrate may also be part of or comprise a particle filter such as a cylindrical container or a rectangular filter cartridge filled with a plurality of particles. In a particle filter, the substrate may comprise a plurality of spheres or beads coated with the active layer and/or the antimicrobial composite. Moreover, the substrate may be part of or comprise a fabric (woven) filter for water or air filtration.

A filtration device according to the invention may comprise, for example, at least two carriers, wherein at least one first carrier is at least in part covered with at least one of graphene and graphene oxide and at least one second carrier is at least in part coated with an antimicrobial composite comprising metallic silver and metallic ruthenium. The carrier may be, for example, a material selected from the group consisting of a sieve, a mesh, a mat, a fleece, a sphere, a bead, and a particle.

For example, the filtration device according to the invention may be part of a carbon filter system. A carbon filter comprises a bed of activated carbon to remove contaminants and impurities such as chlorine, sediment, volatile organic compounds (VOCs), taste and odor from water or air. It is also used in a number of specific applications, including respirator masks. Due to its huge surface area, allowing contaminants the maximum possible exposure to the active sites of the carbon particles, activated carbon works via adsorption, whereby pollutant molecules in the fluid to be treated are trapped inside the pore structure of the carbon particles. Typical particle sizes that can be removed by carbon filters range from 0.5 to 50 µm. However, such carbon filter systems are susceptible to bacterial contamination since the bacteria can easily adhere to the surface of the carbon particles. In order to avoid such bacterial contamination, the filtration device according to the invention can be integrated into the carbon filter system, either as part of the particle filling or as separate unit disposed upstream of the carbon filter.

In another advantageous and exemplary embodiment of the invention the active layer includes pores, cracks or gaps, so that the filtration performance of the filtration device is not significantly impaired by the active layer.

In a further advantageous and exemplary embodiment of the invention the active layer is chemically modified in order to enhance its capturing function. For example, the active layer may comprise modified graphene oxide ("GOX") wherein the 2D graphene oxide sheets are functionalized by specific groups. Basically, the active layer can be at least partly functionalized with at least one compound selected from the group consisting of a polymer (e.g., poly(trimethylamino)ethyl methacrylate), a chelating agent, and a macrocycle (e.g., cyclodextrin). For example, the carbon layer may be functionalized with tailored (bio)ligands for more specific applications, i.e., the outbreak of a new type of pathogen for which no vaccines are available. That is, the filtration device according to the invention can be differentiated in a modular fashion by functionalization of the active layer.

Based on the large number of multiple interactions of functional binding moieties (e.g., quaternary ammonium groups) on the carbon sheets (e.g., "GOX" sheets), the functionalized active layer of such embodiments of the filtration device according to the invention can bind to a large variety of pathogens with extremely high affinity so that germs which have been in contact with the carbon material cannot be released anymore. Furthermore, after binding of the germs to the carbon material, the 2D carbon sheet scaffold can interact with the lipid shell of the germs and disrupt the respiratory process, resulting in the death of pathogens and therefore prohibiting their further transmission. That is, if specifically functionalized, the active layer can contribute additional antimicrobial properties to the overall effect of the filtration device according to the invention.

In another advantageous and exemplary embodiment of the invention at least one of the metallic silver and the metallic ruthenium forms a porous or cluster-like layer. In order to enable sufficient contact and interaction between silver and ruthenium, it is advantageous if both metals are in moisture contact with the environment, or can get into moisture contact with the environment, through continuous, preferably finely formed, free surface areas, openings, pores, (micro)cracks, spacings or the like in at least one metal layer.

In another advantageous and exemplary embodiment of the invention the antimicrobial composite additionally comprises a vitamin selected from the group of water-soluble vitamins with antioxidant properties or a salt or acidic derivative of this vitamin. For example, the vitamin may be at least one of ascorbic acid and a derivative of ascorbic acid. Such vitamins enhance the interaction of silver and ruthenium and thereby improve the anti-microbial effect of the antimicrobial composite. Particularly ascorbic acid (or its derivatives) promotes the catalytic and electrochemical processes of the silver/ruthenium system so that more efficient killing of pathogens at the surface of the filtration device according to the invention is achieved.

In addition to the vitamin, at least one surface-active substance may also be applied to the antimicrobial composite in order to further promote the catalytic and electrochemical processes of the silver/ruthenium system. For example, anionic, non-ionic, amphoteric, or cationic surfactants or suitable mixtures thereof may be used. In particular, alkyl ether sulfates, alkyl and/or aryl sulfonates, alkyl sulfates, amphosurfactants, betaines, alkylamidoalkylamines, alkyl-substituted amino acids, alkyl-substituted imino acids, acylated amino acids, and amphosurfactant combinations can be used.

In yet another advantageous and exemplary embodiment of the invention at least a part of at least one of the active layer, the antimicrobial composite and the substrate are modified by means selected from the group consisting of gravity coating, dip coating and urethane linkage. Using such standard methods, the filtration device according to the invention can be easily modified in a cost-effective manner.

Adsorption of microorganisms by the active layer can be realized by modification of graphene or graphene oxide with ionic groups. In a further advantageous and exemplary embodiment of the invention at least one quaternary ammonium cation is associated with the graphene oxide or reduced graphene oxide. For example, quaternary ammonium cations are covalently bound to graphene oxide or reduced graphene oxide ("GOX"). Graphene oxide can be reduced thermally or by any other suitable reductive process. As the isoelectric point of the functional groups on the surface of bacterial cells (both gram-positive and gram-negative) is lower than 7, bacteria exhibit a negative overall charge at their surface in aqueous solutions and under physiological conditions. Providing the active layer with a plurality of positive charges at its surface therefore results in an increased affinity of the active layer to a plurality of different bacteria. Additionally or alternatively, at least a part of at least one of the active layer, the antimicrobial composite and the substrate comprises at least one of a reactive oxygen functional group, an urethane linkage (-NHCO₂-), and a benzophenone group ((C₆H₅)₂CO). Such modifications are especially advantageous for producing porous substrates and suitable for ensuring a reliable production process.

The invention further concerns a method for producing a filtration device, said method comprising:
Providing at least one substrate;
Applying at least one active layer onto the surface of a first substrate, wherein the active layer comprises at least one of graphene and graphene oxide; and Applying at least one antimicrobial composite onto at least a part of the surface of at least one of the first substrate and the active layer or of a second substrate, wherein the antimicrobial composite comprises metallic silver and metallic ruthenium.

In an advantageous and exemplary embodiment of the method according to the invention the antimicrobial composite is applied by spray coating or by dipping, using electroless plating or immersion techniques. Coating of the substrate and/or the active layer can be accomplished, e.g., by applying metallic ruthenium onto a silver-containing surface of the porous substrate, or by applying metallic silver onto the surface of the substrate and/or the active layer and subsequently applying metallic ruthenium onto the surface of the silver layer, or applying ruthenium/silver bimetallic particles to the substrate and/or the active layer. Optionally, ascorbic acid or a derivative of ascorbic acid and/or at least one surface-active substance may be applied to the antimicrobial composite in order to further enhance its anti-microbial effect. The silver and/or ruthenium layer(s) can also be applied or deposited galvanically. Other coating methods, such as PVD, CVD, sputtering, sol-gel and reduction methods, and also plating methods are suitable for this purpose as well.

In another advantageous and exemplary embodiment of the method according to the invention the substrate is prepared for coating by plasma activation (atmospheric or vacuum plasma). Such pre-treatment of the substrate activates its surface and thereby facilitates coating and stabilizes the adhesion of the coating.

In a further advantageous and exemplary embodiment of the method according to the invention a polymer (e.g. polydopamine) is applied by dipping or spraying technologies onto at least one of the substrate and the active layer. Such modification of the substrate and/or the active layer activates its/their surface and thereby facilitates coating and stabilizes the adhesion of the coating.

The invention also concerns a filter system comprising at least two substrates or at least two sides of the same substrate, wherein at least one side of each substrate or each side of the same substrate is an active side that is brought together into direct contact with another active side, wherein at least one active side is at least in part covered with at least one of graphene and graphene oxide and at least one other active side is at least in part coated by a antimicrobial composite, wherein the antimicrobial composite comprises metallic silver and metallic ruthenium.

In an advantageous and exemplary embodiment of the filter system according to the invention the distance between the active sides that are brought together into direct contact is less than 0.1 mm.

The invention further concerns a filter system comprising at least two components, wherein at least one first component comprises at least one of graphene and graphene oxide and at least one second component comprises an antimicrobial composite comprising metallic silver and metallic ruthenium. The first component may either consist of graphene and/or graphene oxide (including their functionalized forms "GOX") or be provided with at least one active layer comprising graphene and/or graphene oxide (including their functionalized forms "GOX"). Likewise, the second component may either consist of the antimicrobial composite or be coated therewith. That is, the first component may comprise either particles made of cross-linked graphene or graphene oxide sheets or carriers provided with at least one active laver. The second component may comprise either particles made of silver and ruthenium (e.g., silver particles coated with ruthenium clusters) or carriers coated with the antimicrobial composite. A carrier may be, for example, a material selected from the group consisting of a sieve, a mesh, a mat, a fleece, a sphere, a bead, and a particle.

The filtration device and filter systems according to the invention have the following advantages compared to prior art techniques:
- Easy application and combination with existing products such as existing air and water filters;
- Broad spectrum of activity against bacteria, viruses, fungi, spurs and even multi-resistant microorganisms;
- No release of poisonous substances for antimicrobial effect (no Ag⁺, Cu⁺⁺ or biocides);
- Not toxic for the environment, i.e. no health risks for people and animals;
- No special handling dressings or storages necessary;
- No energy consumption or heating necessary for disinfection;
- Little weight, i.e. suitable for mobile systems; and
- High flow rates of filter material due to much larger pores compared to microfiltration applications.

The invention is further described in detail with reference to the figures and tables.

### Brief description of the figures

**Figure 1** shows scanning electron microscope (SEM) images of a) uncoated filter paper (cellulose based) and b) filter paper (cellulose based) coated with modified graphene oxide (graphene oxide modified with quaternary ammonium = GOX); c) photograph of the coated filter paper of b).
**Figure 2** shows the determination of the capture ability of modified graphene oxide (GOX) coated filter paper: a) bar diagram demonstrating the quantified capture efficiency determined by a lab-built filtration assay, b) illustration of the designed filtration assay.
**Figure 3** shows photographs of agar plates representing the results of growth inhibition assays after 24 hours incubation of *Escherichia coli* cells (upper photos) and methicillin-resistant *Staphylococcus aureus* (MRSA) cells (lower photos) at 37°C. The experiments were performed by placing the samples and control horizontally on the agar plates, as well as by inserting them vertically into the agar in the plates. The experiments were performed in triplicates and repeated twice. "AGXX" = antimicrobial composite comprising silver (Ag), ruthenium (Ru) and ascorbic acid.
**Figure 4** shows a schematic representation of one exemplary embodiment of a filtration device according to the invention being performed as a depth filter.
**Figure 5** shows bar diagrams representing the immobilization of bacteria by chemically modified graphene oxide ("GOX"); A) Immobilization of *E. coli* using 300 mg GOX compared to standard filter material ("BRITA"-Filter and "Kohle-Compretten^{®}", Merck Selbstmedikation GmbH); B) Immobilization of S. *aureus* using 300 mg GOX compared to standard filter material ("BRITA"-Filter and "Kohle-Compretten^{®}", Merck Selbstmedikation GmbH); C) Immobilization of *E. coli* using different amounts of chemically modified graphene oxide ("GOX").
**Figure 6** shows schematic representations of two exemplary embodiments of the filtration device according to the invention. A) Filter element (cartridge) including coated mats of fibers for air filtration; B) Filter element (cartridge) including coated beads for water filtration.

### Description of advantageous and exemplary embodiments of the invention

Basically, the active layer is a two-dimensional carbon sheet of graphene and/or graphene oxide with attached functional groups ("GOX"). The GOX layer attracts and/or binds cells with extremely high affinity, similar to the attachment of two sticky stripes of a hook-and-loop fastener. Because of this high binding affinity, microorganisms (pathogens, germs), which (passively) contact the GOX material are captured and hence presented to the antimicrobial composite to be destroyed. In particular, GOX may comprise a component consisting of a two-dimensional graphene oxide sheet, which is chemically modified with quaternary ammonium. GOX can be used as filter material, wherein its size can be tuned by cross-linkage based on urethane groups (-NH-(C=O)-O-).

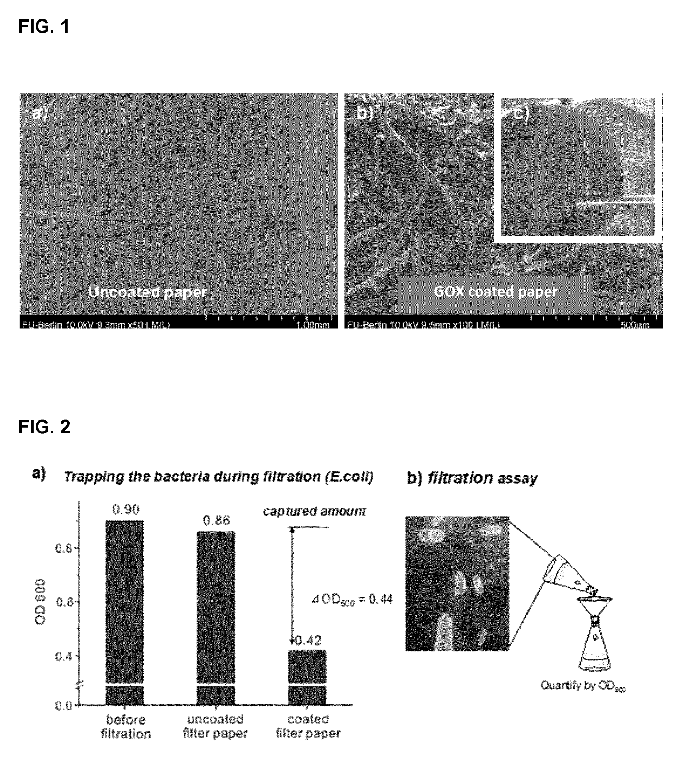

It could be demonstrated that a solution of GOX (graphene oxide functionalized with quaternary ammonium) can immobilize both Gram-negative and Gram-positive bacteria (video evidence from confocal microscopy, not shown) That is, GOX is able to reduce the mobility of *E. coli* (Gram-negative) and *Bacillus subtilis* (Gram-positive) significantly. Moreover, it could be demonstrated that GOX materials can be coated on a common filtration matrix such as cellulose based filter paper (**Figure 1**). Coating of filter paper with GOX can be accomplished, for example, by urethane linkage (-NHCO₂-).

The efficiency of the GOX coated filter paper was quantified using a filtration assay as shown in **Figure 2b****.** Around 50% of bacteria were retained on the GOX coated filter paper (**Figure 2a**). Thus, the GOX coated filter paper can provide the required immobilization effect, i.e. microorganisms can be captured by the modified graphene oxide and presented to the antimicrobial composite.

### Effect of GOX on MRSA

GOX was added to tubes containing the bacterial culture in a growth medium and water, respectively, and incubated at room temperature for 30 minutes. Sterile Milli-Q water was used as a control. The optical density (OD₆₀₀) was measured at the beginning of the experiment and after incubation with GOX. The experiments were performed in triplicates and repeated twice. The difference between OD₆₀₀ before and after incubation with GOX was calculated, and the mean of the difference between OD₆₀₀ values was calculated. The samples after incubation show a clearing of the medium/water as the GOX captures the bacterial load. The effect was stronger with medium than with water as demonstrated visually and photometrically (**Table 1**).

Similar results were obtained for the other bacteria, *E. faecalis* 12030 and *E. coli* BL21 DE3

**Table 1. Effect of GOX on MRSA**

| **Sample** | **Mean of the difference (ΔOD₆₀₀)** |
|---|---|
| Water inoculated with MRSA (before incubation) | 0.90 |
| Water inoculated with MRSA (after incubation) | 0.76 |
| Control | 0.85 |
| Medium inoculated with MRSA (before incubation) | 0.9 |
| Medium inoculated with MRSA (after incubation) | 0.42 |
| Control | 0.86 |

The antimicrobial (Ag/Ru) composite can be disposed onto the porous substrate and/or the active layer, for example, by depositing a silver layer onto the surface of at least one of the porous substrate and the active layer, and subsequently depositing a ruthenium layer onto the silver layer. At least one of the metal layers (e.g., the ruthenium layer) may be accomplished porous or cluster-like so as to ensure sufficient contact and interaction between silver and ruthenium. To this end, one or both metal layer(s) may comprise continuous, preferably finely formed, free surface areas, openings, pores, (micro-)cracks, spacings or the like. The Ag/Ru layers may additionally be provided with a thin layer of ascorbic acid and optionally detergents. For example, a solution comprising 100 mM ascorbic acid, 0.3 % SDS, and 0.2 % Tween 20 can be applied to the surfaces of the Ag/Ru layers by quick immersion, allowing the excess to drip off, and drying. However, the addition of a surface active compound is not necessary in any case.

The resulting antimicrobial composite is based on micro-galvanic elements with surface catalytic properties formed by silver and ruthenium. A micro-electric field is generated at the surface of the antimicrobial composite, which causes damage of the cells captured by the active layer. In particular, reactive oxygen species (ROS) generated by catalytic reduction of O₂ at micro-cathodes and the transition of electrons from the cells to micro-anodes are destroying infectious microorganisms. That is, the antimicrobial effect of the antimicrobial composite is a catalytically supported physico-chemical process based on a direct interaction of the microorganisms with the redox-active centers of micro-cathodes and micro-anodes at the composite's surface. It has been demonstrated that more than 80 different germs (i.e. antibiotic resistant germs, *E. coli,* coliform bacteria, *Staphylococcus aureus, Pseudomonas aeruginosa, Enterococcus faecium, Legionella,* etc.) can be efficiently destroyed by the antimicrobial composite and this composite is also a very efficient biofilm inhibitor nearly independent of biofilm forming species (Guridi et al., 2015; Grohmann et al., 2015).

*Staphylococcus aureus* is a part of the normal skin flora of the human body. It is also often found in the respiratory tract and commonly causes skin and respiratory tract infections. *S*. *aureus* resistant to β-lactam antibiotics, specifically resistant to methicillin, are termed as Methicillin-resistant *Staphylococcus aureus* (MRSA). Enterococci are nosocomial pathogens capable of causing various infections in the human body, such as in surgical areas and the blood stream. The most abundant species is *Enterococcus faecalis.* Both of these bacteria are able to build strong biofilms on surfaces, making them more resistant to antibiotic treatment, physical and chemical stresses. Since biofilm associated and multiple drug resistant bacterial infections are very difficult to treat with antibiotics, it calls for alternative antimicrobial agents or methods to treat these infections.

The antimicrobial composite of the filtration device according to the invention was tested for its efficiency to act as an antibacterial agent against strong biofilm forming clinical MRSA and *E. faecalis* strains. Experimental data for the antibacterial effect of the antimicrobial composite, GOX, and both of the nosocomial pathogens *E. faecalis* and MRSA are presented.

Experiments were performed to investigate the effect of GOX and Ag/Ru on S. *aureus* (MRSA) (Strain 04-02981: Nübel et al., 2010), *E. faecalis* 12030 (biofilm forming Gram-positive bacteria, clinical isolate) and *E. coli* BL21 DE3 (Gram-negative bacteria, lab strain). Experiments in solid and liquid media were performed to examine the capturing efficiency and the anti-bacterial growth inhibitory effect of GOX and Ag/Ru, respectively on the MRSA strain *S*. *aureus* 04-02981, *E. faecalis* 12030 and *E. coli* BL21 DE3. Filter experiments with GOX powder as well as filters coated with GOX were carried out. All the experiments were performed in triplicates and repeated thrice.

### Growth Inhibition Assay

Different antimicrobial materials and proper controls were tested, namely V2A (stainless steel), V2A-Ag, V2A-Ag/Ru, fleece, fleece + Ag/Ru, GOX, individually, as well as in combinations, by placing them horizontally on an agar plate as well as vertically inserting them into the plate. The size of each material used for the assay was 0.25 cm². The assay was performed as per CLSI (The Clinical and Laboratory Standards Institute) guidelines for disk diffusion susceptibility testing (Naas et al., 2006). *S*. *aureus* 04-02981, *E. faecalis* 12030 and *E. coli* BL21 DE3 were investigated. The plates were incubated at 37°C, monitored after 24 h (**Tables 2 and 3**), 48 h, 72 h, 96 h and 120 h.

V2A- Ag/Ru, V2A-Ag and V2A meshes used were tested with two different mesh widths, 200µm and 50µm. The experiments were performed in triplicates and repeated twice. The mean of the values was calculated. From the results obtained, no zone of inhibition was observed in case of V2A-Ag, and the controls V2A and fleece, samples with mesh width 200µm showed larger zones of inhibition than samples with mesh width 50µm (**Table 2**).

Fleece + Ag/Ru, Fleece +GOX, and Fleece + GOX + Ag/Ru were used to check their effect on the bacteria, fleece was used as a negative control. The experiments were performed by placing the samples and controls horizontally on agar plates, as well as by inserting them vertically into the agar in the plates. The experiments were performed in triplicates and repeated twice. The mean of the values was calculated. From the results obtained, no zone of inhibition was observed in case of the control fleece and fleece + GOX. Fleece + GOX + Ag/Ru and fleece + Ag/Ru showed similar zones of inhibition (**Table 3**).

**Table 2. Effect of Ag/Ru (coated on different surfaces) and silver on bacteria**

| **Bacteria** | **Sample** | **Inhibition zone [cm]** |
|---|---|---|
| *S*. *aureus* 04-02981 | Fleece + Ag/Ru | 1.8 |
| | Control (fleece) | No inhibition |
| | V2A- Ag/Ru 200µm | 0.9 |
| | V2A-Ag | No inhibition |
| | V2A | No inhibition |
| | V2A- Ag/Ru 50µm | 0.7 |
| | V2A-Ag | No inhibition |
| | V2A | No inhibition |
| | | |
| *E. faecalis* 12030 | Fleece + Ag/Ru | 1.6 |
| | Control (fleece) | No inhibition |
| | V2A- Ag/Ru 200µm | 0.6 |
| | V2A-Ag | No inhibition |
| | V2A | No inhibition |
| | V2A- Ag/Ru 50µm | 0.5 |
| | V2A-Ag | No inhibition |
| | V2A | No inhibition |
| | | |
| *E. coli* BL21 DE3 | Fleece + Ag/Ru | 1.9 |
| | Control (fleece) | No inhibition |
| | V2A- Ag/Ru 200µm | 1.1 |
| | V2A-Ag 200µm | No inhibition |
| | V2A 200µm | No inhibition |
| | V2A- Ag/Ru 50µm | 0.9 |
| | V2A-Ag 50µm | No inhibition |
| | V2A 50µm | No inhibition |

**Table 3. Effect of fleece + Ag/Ru and GOX on bacteria**

| **Bacteria** | **Sample** | **Inhibition zone [cm]** |
|---|---|---|
| *S*. *aureus* 04-02981 | Fleece | no inhibition |
| | Fleece + GOX | no inhibition |
| | Fleece + GOX + Ag/Ru | 1.2 |
| | Fleece + Ag/Ru | 1.3 |
| *E. coli* BL21 DE3 | Fleece | no inhibition |
| | Fleece + GOX | no inhibition |
| | Fleece + GOX + Ag/Ru | 1.4 |
| | Fleece + Ag/Ru | 1.4 |
| *E. faecalis* 12030 | Fleece | no inhibition |
| | Fleece + GOX | no inhibition |
| | Fleece + GOX + Ag/Ru | 1.1 |
| | Fleece + Ag/Ru | 1.5 |

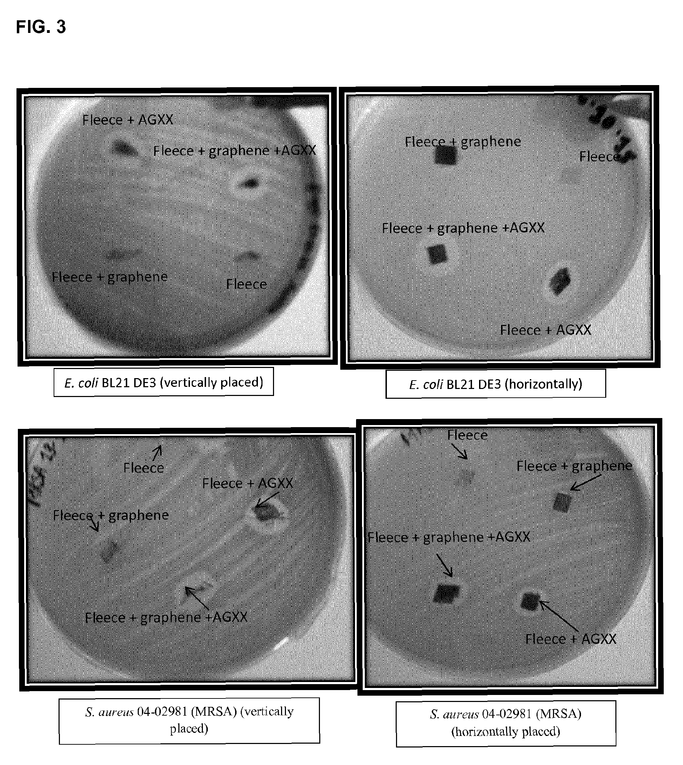

The effect of Fleece, Fleece + Ag/Ru, Fleece + GOX and Fleece + GOX + Ag/Ru on *S*. *aureus* 04-02981, *E. faecalis* 12030 (data not shown) and *E. coli* BL21 DE3 was tested individually and in combination (**Figure 3**). The bioactive composite (Ag/Ru) is a contact catalyst on the basis of oxidation-reduction potential producing reactive oxygen species (ROS) that kill bacteria. Therefore, the filtration device according to the invention is designed such that in combination with GOX the ROS are still generated and the intrinsic antimicrobial property of Ag/Ru is not inhibited by GOX. To verify so, a combination of an Ag/Ru coated fleece (see its antimicrobial effect in **Figure 3**) and a GOX coated filter paper was provided. When this Ag/Ru and GOX combination was used in a filtration assay as described above, the antimicrobial property of Ag/Ru was retained. As shown in **Figure 3**, the growth inhibition zone is clearly visible when GOX and Ag/Ru are combined. Accordingly, the filtration device according to the invention allows for effective capturing and killing of microorganisms that are retained by the filtration device, wherein the microorganisms are captured by the active layer (GOX) and then destroyed by the antimicrobial composite (silver and ruthenium).

For example, the filtration device according to the invention may be performed as a depth filter that uses a porous filtration medium to retain particles throughout the medium, rather than just on its surface. Depth filters often comprise multiple porous layers capable of capturing solid contaminants from a fluid (liquid or gas). Depth filters are usually used if a fluid to be filtered contains a high load of particles because, relative to other types of filters, they can retain a large mass of particles before becoming clogged.

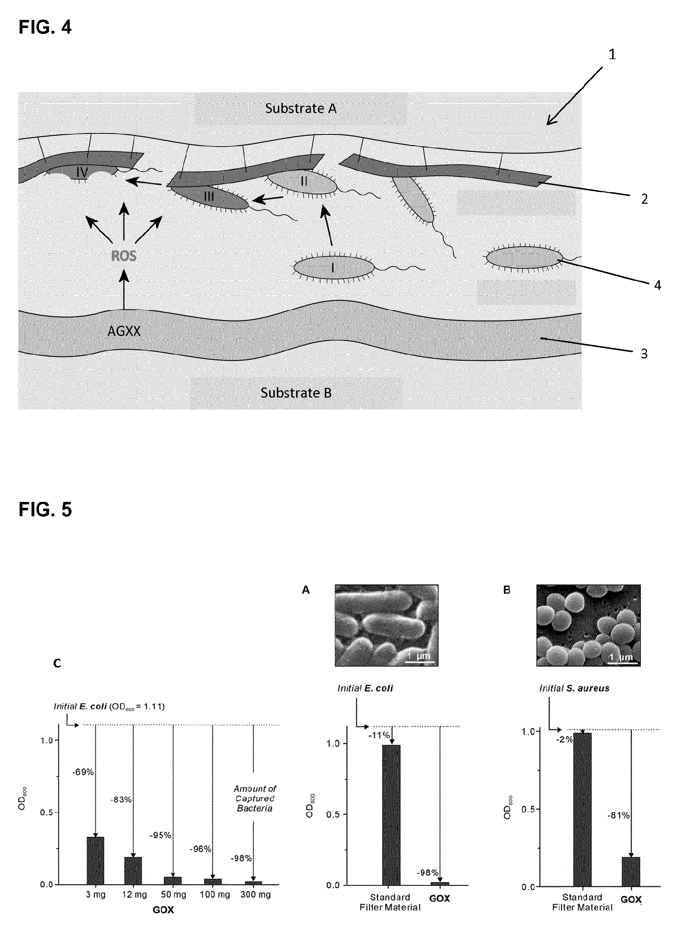

As shown in **Figure 4****,** the filtration device 1 may comprise two substrates, substrate A being provided with a chemically functionalized graphene or graphene oxide layer (active layer 2) and substrate B being coated with an antimicrobial composite 3 comprising silver and ruthenium ("AGXX"). The active layer 2 comprises graphene or graphene oxide functionalized by ionic groups so that it can attract microorganisms 4 and endotoxins (Step I), which are then captured and bound by the active layer 2 (Step II). The antimicrobial composite 3 produces and releases reactive oxygen species ("ROS") which kill microorganisms present in the surroundings (Step III). As a certain retention time is necessary for this process, it is important that the microorganisms are captured by the active layer 2 and thus exposed to the antimicrobial products of the antimicrobial composite 3. As the cell structure of the microorganisms is destroyed during the killing process, the cell debris is at least in part removed (purged) from the active layer 2 (Step IV). Due to this beneficial regeneration of the active layer 2, capacity and durability of the filtration device 1 is significantly increased.

As shown in **Figure 5****,** a 95% reduction of the optical density of an *E. coli* suspension can be achieved with only 50 mg chemically modified graphene oxide (GOX). Addition of 300 mg GOX results in a 98% reduction of bacteria concentration (C). Accordingly, it can be observed that GOX is capable of capturing bacteria. Compared to the same amount of standard filter material, GOX provides a significantly increased reduction of bacteria concentration (A and B). Moreover, it can be observed that bacteria immobilized on the GOX surface are not able to proliferate. That is, in addition to the capturing property, GOX provides a significant growth inhibitory effect.

### Maximum bacterial intake capacity of GOX material: at least 6.4 x10¹⁰ CFU/g (CFU = colony-forming units)

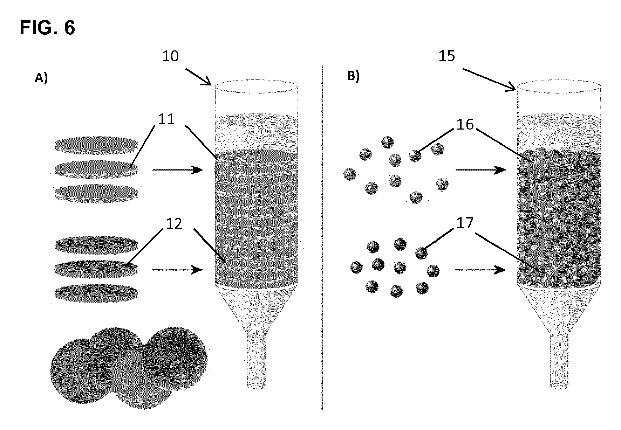

**Figure 6** shows two exemplary embodiments of a filtration device 10, 15. The first filtration device 10 is a filter element (cartridge) for filtration of air or other gas (A). This device 10 is filled with two types of mats of fibers. The first type of mats is coated with an antimicrobial composite 11 comprising metallic silver and metallic ruthenium while the second type of mats is provided with active layers 12 comprising graphene or graphene oxide. The second filtration device 15 is a filter element (cartridge) for filtration of water or other liquids (B). This device 15 is filled with two types of beads. The first type of beads is coated with an antimicrobial composite 16 comprising metallic silver and metallic ruthenium while the second type of beads is provided with active layers 17 comprising graphene or graphene oxide.

### References

A. Guridi, A.-K. Diederich, S. Aguila-Arcos, M. Garcia-Moreno, R. Blasi, M. Broszat, W. Schmieder, E. Clauss-Lendzian, T. Sakinc-Gueler, R. Andrade, I. Alkorta, C. Meyer, U. Landau, E. Grohmann. A new antimicrobial contact catalyst killing antibiotic resistant clinical and waterborne pathogens. Mat. Science Engin. C, 50, 1-11, 2015.
E. Grohmann, C. Meyer, U. Landau. Der Kontakt-Katalysator AGXX tötet multiresistente klinische Staphylokokken und Enterokokken. Orthopaedic dynamic, Special MRSA. 62-64, 2015.
U. Nübel, J. Dordel, K. Kurt, B. Strommenger, H. Westh, S. Shukla, H. Kova, R. Leblois, T. Wirth, T. Jombart, F. Balloux, W. Witte. A timescale for evolution, population expansion, and spatial spread of an emerging clone of methicillin resistant Staphylococcus aureus. PLOS PATHOGENS. 6(4), 2010.
T. Naas, B. Coignard, A. Carbonne, K. Blanckaert, O. Bajolet, C. Bernet, X. Verdeil, P. Astagneau, J.C. Desenclos, P. Nordmann, French Nosocomial Infection Early Warning Investigation and Surveillance Network, VEB-1 extended-spectrum beta lactamase-producing Acinetobacter baumannii, France, Emerg. Infect. Dis. 12, 1214-1222, 2006.
Abdullahil Kafy, Kishor Kumar Sadasivuni, Hyun-Chan Kim, Asma Akther and Jaehwan Kim. Designing flexible energy and memory storage materials using cellulose modified graphene oxide nanocomposites. Phys.Chem.Chem.Phys., 17, 5923.
Kishor Kumar Sadasivuni, Abdullahil Kafy, Lindong Zhai, Hyun-U. Ko, Seongcheol Mun, and Jaehwan Kim. Transparent and Flexible Cellulose Nanocrystal/Reduced Graphene Oxide Film for Proximity Sensing. small 2015, 11, 8, 994-1002.

## Claims

1. A filtration device (1, 10, 15) comprising:
At least one active layer (2, 12, 17) comprising at least one of graphene and graphene oxide; and
At least one antimicrobial composite (3, 11, 16) comprising metallic silver and metallic ruthenium.

2. The filtration device of claim 1, further comprising at least one substrate.

3. The filtration device of claim 2, wherein the substrate is a porous substrate and/or comprises at least one material selected from the group consisting of a fabric, a polymer, a ceramic, and a metal.

4. The filtration device of claim 2 or 3, wherein at least one of the active layer (2, 12, 17) and the antimicrobial composite (3, 11, 16) is disposed on at least a part of the substrate.

5. The filtration device of any one of claims 1 to 4, wherein the active layer (2, 12, 17) includes pores or gaps.

6. The filtration device of any one of claims 1 to 5, wherein the active layer (2, 12, 17) is chemically modified and/or at least partly functionalized with at least one compound selected from the group consisting of a polymer, a chelating agent, and a macrocycle.

7. The filtration device of any one of claims 1 to 6, wherein at least one of the metallic silver and the metallic ruthenium forms a porous or cluster-like layer.

8. The filtration device of any one of claims 1 to 7, wherein the antimicrobial composite (3, 11, 16) additionally comprises a vitamin selected from the group of water-soluble vitamins with antioxidant properties or a salt or acidic derivative of this vitamin, or wherein the antimicrobial composite (3, 11, 16) additionally comprises at least one of ascorbic acid and a derivative of ascorbic acid.

9. The filtration device of any one of claims 1 to 8, wherein at least a part of at least one of the active layer (2, 12, 17), the antimicrobial composite (3, 11, 16) and the substrate is modified by means selected from the group consisting of gravity coating, dip-coating and urethane linkage.

10. The filtration device of any one of claims 1 to 9, wherein at least one quaternary ammonium cation is associated with the graphene oxide or reduced graphene oxide, and/or wherein at least a part of at least one of the active layer (2, 12, 17), the antimicrobial composite (3, 11, 16) and the substrate comprises at least one of a reactive oxygen functional group, an urethane linkage, and a benzophenone group.

11. A method for producing a filtration device, said method comprising:
Providing at least one substrate;
Applying at least one active layer onto the surface of a first substrate, wherein the active layer comprises at least one of graphene and graphene oxide; and
Applying at least one antimicrobial composite onto at least a part of the surface of at least one of the first substrate and the active layer or of a second substrate, wherein the antimicrobial composite comprises metallic silver and metallic ruthenium.

12. The method of claim 11, wherein the antimicrobial composite is applied by spray coating or by dipping, using electroless plating or immersion techniques.

13. The method of claim 11 or 12, wherein the substrate is prepared for coating by plasma activation and/or wherein a polymer is applied by dipping or spraying technologies onto at least one of the substrate and the active layer.

14. A filter system comprising at least two substrates or at least two sides of the same substrate, wherein at least one side of each substrate or each side of the same substrate is an active side that is brought together into direct contact with another active side, wherein at least one active side is at least in part covered with at least one of graphene and graphene oxide and at least one other active side is at least in part coated by a antimicrobial composite, wherein the antimicrobial composite comprises metallic silver and metallic ruthenium.

15. A filter system comprising at least two components, wherein at least one first component comprises at least one of graphene and graphene oxide and at least one second component comprises an antimicrobial composite comprising metallic silver and metallic ruthenium.
